# EUROPEAN PATENT SPECIFICATION

(11) **EP 2 623 050 B1**
(45) Date of publication and mention of the grant of the patent: **26.11.2014**
(21) Application number: 13151417.6
(22) Date of filing: 16.01.2013
(51) Int. Cl.: A61B 17/16

(54) **Instrument for use in shoulder arthroplasty**
Instrument für die Verwendung bei der Schulterarthroplastie
Instrument destiné à être utilisé en arthroplastie de l'épaule

(30) Priority: 01.02.2012 US 201213363583
(43) Date of publication of application: 07.08.2013
(73) Proprietor: DePuy Synthes Products, LLC, Raynham, MA 02767-0350 (US)
(72) Inventor: Lappin, Kyle, Warsaw, IN Indiana 46581 (US)
(74) Representative: Belcher, Simon James

(56) References cited:
- WO-A1-2011/005205
- CH-A5- 693 446
- US-A- 6 045 302
- US-A1- 2004 097 947

## Description

The present invention relates generally to the field of orthopaedics, and, more particularly, to an instrument for use in shoulder arthroplasty.

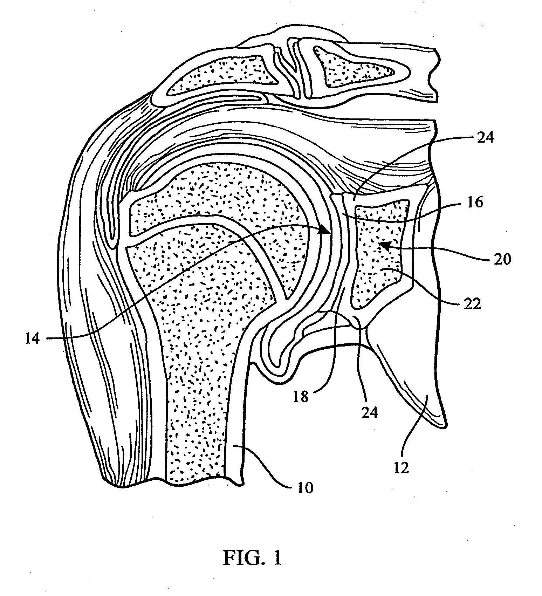

As shown in FIG. 1, a typical shoulder or glenohumeral joint is formed in a human body where the humerus 10 movably contacts the scapula 12. The scapula 12 includes a glenoid fossa 14 that forms a socket against which the head of the humerus 10 articulates. At this socket, the scapula 12 includes cartilage 16 that facilitates such articulation. Beneath the cartilage is subchondral bone 18 that forms a wall of a glenoid vault 20 that defines a cavity which contains cancellous bone 22. The subchondral bone 18 that forms the glenoid vault 20 defines a glenoid rim 24 at a periphery of the glenoid vault 20 that is attached to the cartilage 16. During the lifetime of a patient, the glenoid fossa 14 may become worn, especially at its posterior and/or superior portions thereby causing severe shoulder pain and limiting the range of motion of the patient's shoulder joint. To alleviate such pain and increase the patient's range of motion, a shoulder arthroplasty may be performed. Arthroplasty is the surgical replacement of one or more bone structures of a joint with one or more prostheses.

Shoulder arthroplasty often involves replacement of the glenoid fossa of the scapula with a prosthetic glenoid component. The conventional glenoid component typically provides a generally laterally or outwardly facing generally concave bearing surface against which a prosthetic humeral head (or, alternatively, the spared natural humeral head in the case of a glenoid hemi-arthroplasty) may bear during operation of the joint. The conventional glenoid component typically also includes a generally medially or inwardly projecting stem for fixing the glenoid component in a cavity constructed by suitably resecting the glenoid fossa 14 and suitably resecting cancellous bone 22 from the glenoid vault 20.

The goal of shoulder arthroplasty is to restore normal kinematics to the shoulder. Accordingly, known systems attempt to replicate the normal kinematics by carefully controlling the geometry of the articulating surfaces in the joint as well as the positioning of the prostheses in the bones in which the prostheses are implanted. Thus, the articulating surface of a humeral component is typically spherical and positioning of the humeral component is accomplished by using the anatomical neck of the humerus as the reference plane for reconstruction of the humeral head.

Traditionally, shoulder joints have been understood to exhibit translation of the humeral component on the glenoid component in addition to rotation. Thus, the articulating surface of the glenoid is typically formed with a radius of curvature that is much larger than the radius of curvature of the humeral component. The increased radius of curvature of the glenoid articulating surface can be from 2 to 6 mm larger than the radius of curvature for the humeral component in these systems.

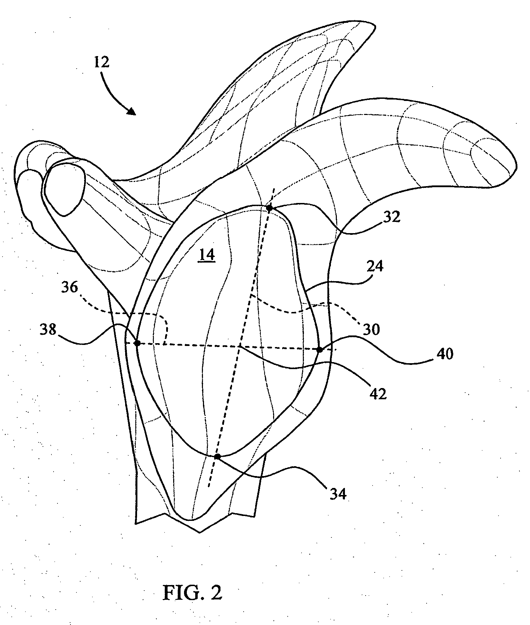

In known systems, the glenoid component is positioned in the geometric centre of the glenoid fossa. The geometric centre is established by generating a line from the most superior point of the glenoid rim to the most inferior point of the glenoid rim ("Saller's line"). A second line is generated between the most posterior point of the glenoid rim and the most anterior point of the glenoid rim. The intersection of the two generated lines is considered to be the geometric centre of the area circumscribed by the glenoid rim. By way of example, FIG. 2 shows a sagittal view of the scapula 12. In FIG. 2, Saller's line 30 extends between the most superior point 32 of the glenoid rim 24 to the most inferior point 34 of the glenoid rim 24. A second line 36 extends from the most posterior point 38 of the glenoid rim 24 and the most anterior point 40 of the glenoid rim. The geometric centre 42 of the glenoid fossa 14 is located at the intersection of the line 36 and Saller's line 30. As used herein, the terms anterior, posterior, superior, and inferior, unless otherwise specifically described, are used with respect to the orientation of the scapula 12 as shown in FIG. 2.

Once a surgeon determines the placement of the glenoid component, a guide pin is positioned through the glenoid fossa. A reamer is then used to shape the scapula to receive a glenoid component, typically by forming a cavity in the glenoid vault. For glenoid components including a centre peg for fixation of the glenoid component within the glenoid vault, a bore is drilled using the guide pin as a guide. The guide pin is then removed. For glenoid components including offset pegs in addition to the centre peg for fixation of the glenoid component within the glenoid vault, a drill guide is introduced into the prepared cavity and additional bores are drilled for each of the offset pegs. A trial glenoid component is then implanted in the prepared cavity and, if the fit appears to be satisfactory, the trial is removed and a glenoid component is implanted in the prepared cavity.

CH-693446 discloses a handtool for transmitting rotational drive to a driver tip, the handtool having a first portion for connection to a rotational drive and a second portion which has a tip for engaging a screwdriver bit. The handtool includes a universal joint transmission connection between the first and second portions.

There exists a need for reducing the instrumentation required to properly prepare the scapula to receive a glenoid component, allowing simplification of methods for shoulder joint replacement.

The invention provides an instrumentation kit for use in preparing a bone to receive a prosthetic component, as defined in claim 1.

The kit can be used in a method of preparing a shoulder to receive a glenoid component which includes accessing a glenoid of a shoulder, rotationally coupling a distal portion of a combination device to an instrument, applying a torque to a proximal portion of the combination device, transferring the applied torque from the proximal portion to the distal portion through a pivoting connection, rotating the instrument using the transferred applied torque, and reaming a portion of the glenoid with a rotating reaming section of the instrument using the transferred applied torque.

Optionally, the method includes pivoting the proximal portion with respect to the distal portion after reaming the portion of the glenoid, rotating the instrument using the pivoted proximal portion, and forming a bore in the glenoid using a first drill guide defined by the instrument.

Optionally, the step of rotationally coupling the combination device and the instrument comprises:
positioning a guide wire in the shoulder,
inserting the guide wire into a first guide bore in the instrument, and
inserting the guide wire into a second guide bore in the distal portion.

Optionally, the step of rotationally coupling the combination device and the instrument involves inserting the guide wire into a third guide bore in the proximal portion.

Optionally, the step of pivoting the proximal portion with respect to the distal portion involves moving the proximal portion away from the instrument so that the guide wire is no longer within the third guide bore.

Optionally, the step of pivoting the proximal portion with respect to the distal portion includes pivoting the proximal portion about a pin extending through a first tine of the proximal portion and a second tine of the distal portion.

Optionally, the step of moving the proximal portion away from the instrument decouples the distal portion of the combination device and the instrument, and the method includes:
moving the distal portion along the guide wire toward the instrument using the pivoted proximal portion,
rotationally aligning the distal portion and the instrument using the pivoted proximal portion, and
recoupling the distal portion and the instrument using the pivoted proximal portion.

Optionally, the method includes rotating the recoupled distal portion and instrument using the pivoted proximal portion.

Embodiments of the invention are described below by way of example with reference to the accompanying drawings, in which:
FIG. 1 is a coronal view of an anatomically normal shoulder joint.
FIG. 2 is a sagittal view of the shoulder joint of FIG. 1.
FIG. 3 is a bottom perspective view of a circular glenoid component that may be implanted in a scapula.
FIG. 4 is a bottom plan view of the circular glenoid component of FIG. 3.
FIG. 5 is a side plan view of the circular glenoid component of FIG. 3.
FIG. 6 is a side perspective view of a combination reamer/drill device that may be used to simultaneously form a bore for receiving a peg of a glenoid component and to ream a glenoid fossa to receive the glenoid component.
FIG. 7 is a bottom perspective view of the combination device of FIG. 6 showing the guide bore extending from a distal tip of the combination device.
FIG. 8 is a bottom plan view of the combination device of FIG. 6.
FIG. 9 is a side plan view of the combination device of FIG. 6 showing the guide bore extending to a drive section which in this device is a hexagonally shaped bore in a body section of the combination device.
FIG. 10 is a perspective view of a combination power extension/anti-rotation handle which may be used with the combination device of FIG. 6 to couple a power rotary tool (not shown) to the combination device.
FIG. 11 is a bottom plan view of the combination power extension/anti-rotation handle of FIG. 10 showing a guide bore which extends from a distal tip of the lower portion.
FIG. 12 is a side cross sectional view of the combination power extension/anti-rotation handle of FIG. 10.
FIG. 13 is a flowchart showing steps in medical procedure that may be used to implant the circular glenoid component of FIG. 3 into a scapula using the combination device of FIG. 10.
FIG. 14 is a perspective view of the scapula of FIG. 2 with a guide wire positioned in the scapula using a guide template and a guide template manipulator.
FIG. 15 is a partial cross-sectional view of the scapula of FIG. 14 with the combination reamer/drill device of FIG. 6 guided to a location adjacent to the glenoid fossa by the guide wire of FIG. 14.
FIG. 16 is a partial cross-sectional view of the scapula of FIG. 14 with the combination power extension/anti-rotation handle of FIG. 10 coupled to the combination reamer/drill device of FIG. 6, both the combination power extension/anti-rotation handle and the combination reamer/drill device guided to a location adjacent to the glenoid fossa by the guide wire of FIG. 14.
FIG. 17 is a partial cross-sectional view of the scapula of FIG. 14 and the coupled combination power extension/anti-rotation handle and the combination reamer/drill devices of FIG. 16 after the combination reamer/drill device has been used to form a bore in preparation for implanting the glenoid component of FIG.3 into the scapula.
FIG. 18 is a partial cross-sectional view of the scapula of FIG. 14 and the coupled combination power extension/anti-rotation handle and the combination reamer/drill devices of FIG. 16 after the combination reamer/drill device has been used to simultaneously ream the glenoid fossa and form a bore in preparation for implanting the glenoid component of FIG.3 into the scapula.
FIG. 19 is a perspective view of the scapula of FIG. 14 after the combination power extension/anti-rotation handle has been withdrawn along the guide wire 236 to a location that allows pivoting of the proximal portion of the combination power extension/anti-rotation handle.
FIG. 20 is a perspective view of the combination power extension/anti-rotation handle after the proximal portion of the combination power extension/anti-rotation handle has been pivoted.
FIG. 21 is a perspective view of the scapula of FIG. 14 after the pivoted combination power extension/anti-rotation handle of FIG. 20 has been guided by the guide wire of FIG. 14 and coupled with the combination reamer/drill device of FIG. 6 allowing a user to manually orient the combination reamer/drill device on the scapula.
FIG. 22 is a perspective view of the scapula of FIG. 14 with the combination reamer/drill device of FIG. 6 used to guide a drill bit to form a bore to receive an offset peg of the glenoid component of FIG. 3 while the pivoted combination power extension/anti-rotation handle of FIG. 20 has been used to manually stabilize the combination reamer/drill device on the scapula.
FIG. 23 is a perspective view of the combination reamer/drill device of FIG. 6 and drill bit of FIG. 22 with the pivoted combination power extension/anti-rotation handle of FIG. 20.

The same reference numerals are used in the drawings to refer to the same parts.

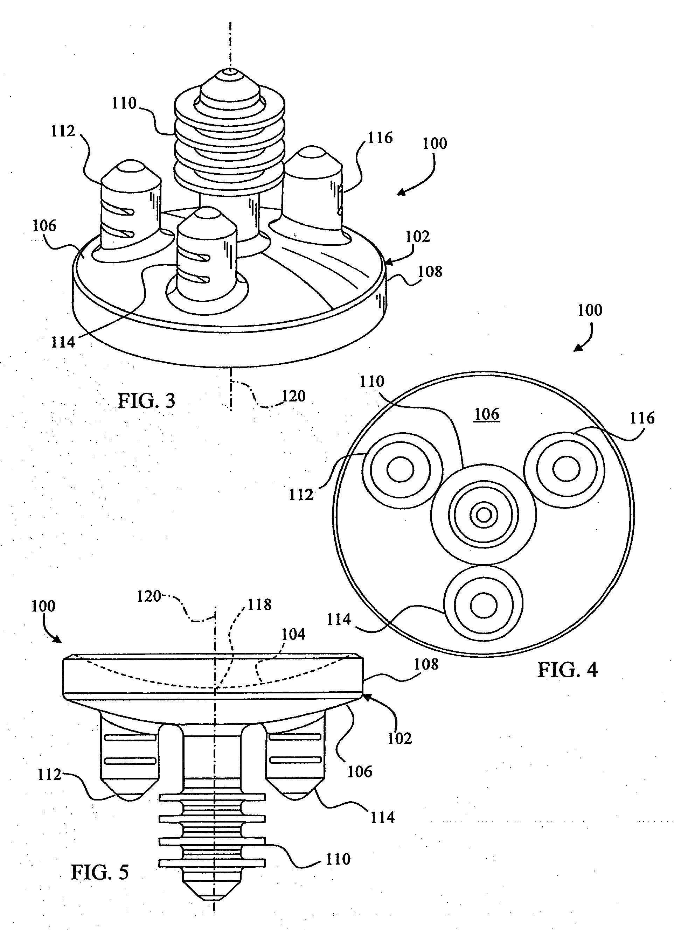

Referring to the drawings, FIGs. 3 to 5 show a glenoid component 100. The glenoid component 100 includes a body portion 102 including a spherical articulating surface 104 and an opposite bone contacting surface 106. An outer wall 108 extends away from the bone contacting surface 106 and defines an outer periphery of the body portion 102. The bone contacting surface 106 is generally convex. A finned centre peg 110 extends away from the nadir of the bone contacting surface 106 as viewed in FIG. 5. Three offset pegs 112, 114, 116 extend away from the bone contacting surface 106 at locations between the centre peg 110 and the outer wall 108. The nadir 118 of the spherical articulating surface 104 is located on the centerline 120 of the glenoid component 100.

The glenoid component 100 is an integrally formed unit made from a durable biocompatible plastic or any other suitable durable biocompatible material. For example, the glenoid component 100 may be made from a polyethylene. One particular polyethylene that is well suited for glenoid component 100 is a high molecular weight polyethylene, for example ultra-high molecular weight polyethylene ("UHMWPE"). Components made from a UHMWPE material are sold by Johnson & Johnson of New Brunswick, New Jersey under the trade mark MARATHON. Details of the material are disclosed in US-6228900 and US-6281264.

In components in which the articulating surface 104 and the other portions of the glenoid component 100 are made from different materials, the portions of the glenoid component 100 other than the articulating surface 104 may be made from a suitable biocompatible metal such as, for example, a cobalt chromium alloy, a stainless steel alloy, a titanium alloy, or any other suitable durable material. In these components, the articulating surface 104 is secured to the body portion 102 in any suitable manner. For example, articulating surface 104 may be bonded to body portion 102, or articulating surface 104 could be made from polyethylene and compression moulded to body portion 102. Alternately, the articulating surface 104 may be glued to the body portion 102 by, for example, an adhesive. Alternatively, articulating surface 104 may be mechanically interlocked to the body portion 102 by taper locking or otherwise press-fitting the articulating surface 104 into the body 102 and the body 102 may include any other suitable interlocking features, for example, rib(s), lip(s), detent(s), and/or other protrusion(s) and mating groove(s), channel(s), or indent(s) (not shown).

In other components, one or more of the outer wall 108, the bone contacting surface 106, the centre peg 110 and the offset pegs 112, 114, 116 may include a porous coating to facilitate bone in-growth into the glenoid component 100. The porous coating may be any suitable porous coating. An example of a suitable porous coating is used in products sold by Johnson & Johnson of New Brunswick, New Jersey under the trade mark POROCOAT. Details of such a coating are disclosed in US-3855638.

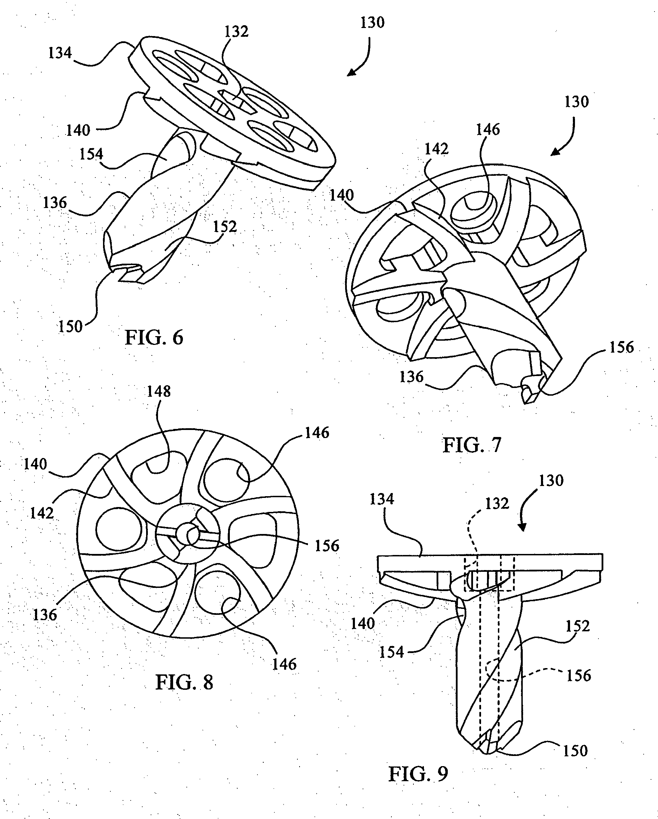

In order to implant the glenoid component 100 into a scapula, the scapula must first be prepared to receive the glenoid component 100. A device which can be used to prepare the scapula to receive the glenoid component 100 is shown in FIGs. 6 to 9. With reference to FIGs. 6 to 9, a combination reamer/drill device 130 includes a drive section 132, a body section 134, and a drill or boring section 136. The drive section 132 in this instrument is a hexagonally shaped bore defined in the body section 134.

A number of reaming fins 140 extend from the lower central portion of the body section 134 toward the drill section 136. The reaming fins 140 curve proximally and outwardly from the lower central portion of the body section 134 to the outer periphery of the body section 134. The reaming fins 140 include an arcuate leading edge 142.The body section 134 defines a number of through-holes at locations between adjacent reaming fins 140. The through-holes in the instrument of FIGs. 6 to 9 include three drill guides 146 and three ports 148.

The drill section 136 extends away from the body section 134 to a distal tip 150. Two flutes 152, 154 extend helically about the drill section 136 between the body section 134 and the distal tip 150. A guide bore 156 extends from the distal tip 150 to the drive section 132.

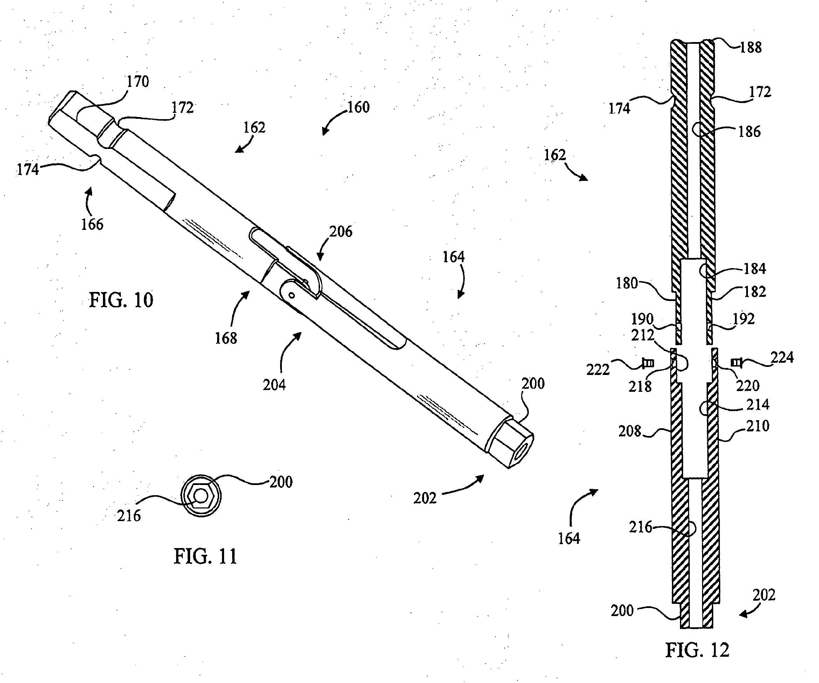

As discussed in further detail below, a kit may include one or more combination reamer/drill devices 130 along with various instrumentation to facilitate use of the combination reamer/drill device 130. FIGs. 10 to 12 show a combination power extension/anti-rotation device 160 which is included in the kit. The combination power extension/anti-rotation device 160 includes a longitudinally extending proximal portion 162 and a longitudinally extending distal portion 164. The proximal portion 162 includes a power receiving portion 166 and a junction portion 168. The power receiving portion 166 is sized and configured to couple with a power tool (not shown) and includes a pair of opposing power receiving flats 170 and a pair of coupling grooves 172 and 174 which extend about the power receiving portion 166 between the power receiving flats 170.

The junction portion 168 includes two tines 180, 182 which define a receiving area 184 between them. A guide bore 186 extends from the receiving area 184 to the proximal tip 188 of the proximal portion 162. Two bores 190, 192 extend through the respective tines 180, 182.

The distal portion 164 includes a power transfer portion 200 at a distal end 202. The power transfer portion 200 is shaped to be complimentary to the drive section 132 of the combination reamer/drill 130. In the instrument of FIGs. 10 to 12, the power transfer portion 200 is thus a hexagonally shaped protrusion sized to fit within the drive section 132.

A junction portion 204 is located at a proximal end 206 of the distal portion 164. The junction portion 204 includes two tines 208, 210 which define an upper receiving area 212 and a lower receiving area 214 between them. A guide bore 216 extends from the lower receiving area 214 to the distal end 202 of the distal portion 164. Two bores 218, 220 extend through the respective tines 208, 210. The bores 218, 220 are countersunk so that two pins 222, 224 may be received therein and be flush with the outer surface of the tines 208, 210.

When the combination power extension/anti-rotation handle 160 is assembled, the tines 180, 182 of the proximal portion 162 are received with in the upper receiving area 212 of the distal portion 162. Additionally, the bores 190, 192 are aligned with the bores 218, 220, respectively. The pin 222 is positioned within the aligned bores 190, 218, while the pin 224 is positioned within the aligned bores 192, 220. The pins 222, 224 and bores 190, 192, 218, 220 are configured to allow the proximal portion 162 to pivot with respect to the distal portion 164 about an axis defined by the pins 222, 224. To this end, the pins 222, 224 in one embodiment are in the form of rivets. In another embodiment, the pins 222, 224 are threadedly engaged with the bores 190, 192, respectively and configured to articulate with the bores 218, 220.

Additionally, the guide bore 186 and the guide bore 216 lie within the same plane when the combination power extension/anti-rotation handle 160 is assembled. As the proximal portion 162 is pivoted with respect to the distal portion 164, the guide bore 186 pivots within that same plane. Accordingly, the guide bores 186, 216 may be pivoted into alignment with each other. When the guide bores 186, 216 are aligned, the proximal portion 162 and the distal portion 164 are longitudinally aligned as shown in FIG. 10.

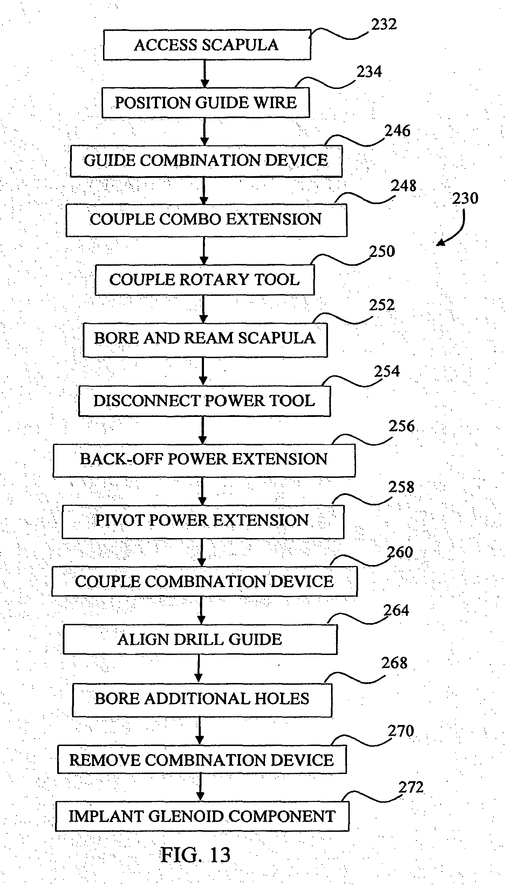

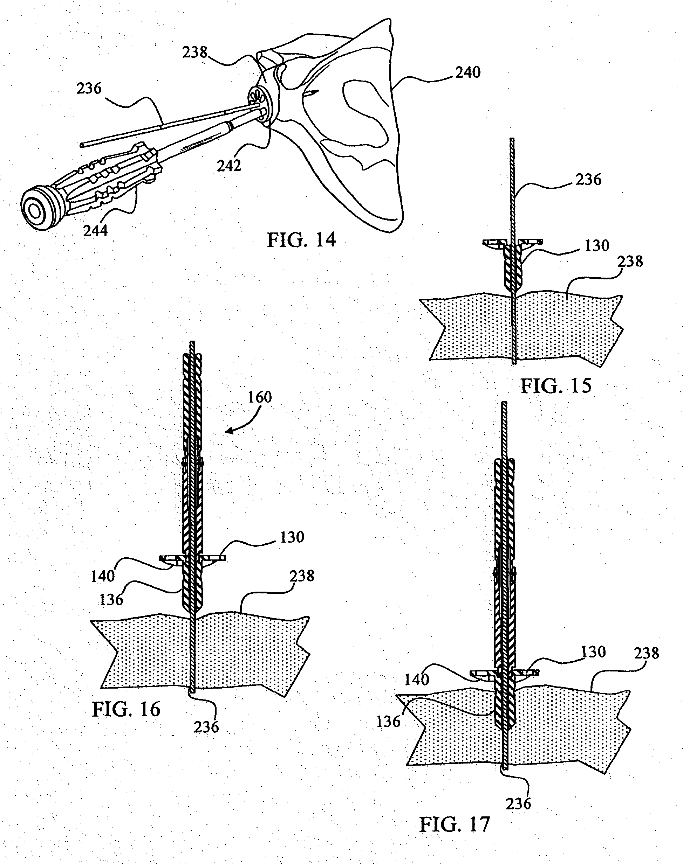

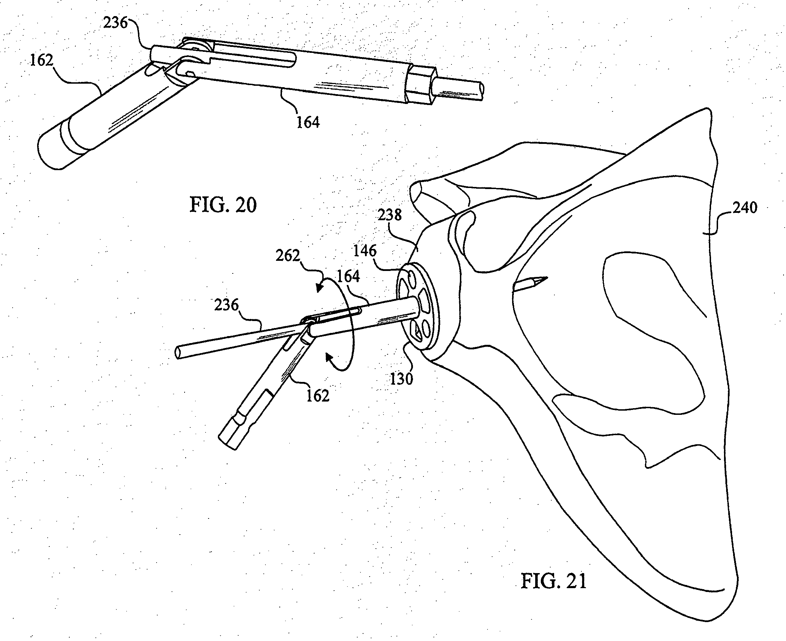

A kit including the combination reamer/drill device 130 and the power extension/anti-rotation handle 160 may be used in preparing a shoulder to receive a glenoid component such as glenoid component 100 in accordance with a procedure 230 shown in FIG. 13. Initially, a scapula is accessed at block 232 in accordance with a desired surgical approach. At block 234, a guide wire, which is provided in the kit along with other instrumentation used in the procedure 230, is positioned on the scapula. Positioning of the guide wire may be computer aided. Optionally, the guide wire is positioned based upon identification of the centre of an inferior glenoid circle. By way of example, FIG. 14 shows a guide wire 236 implanted into a glenoid 238 of a scapula 240. FIG. 20 shows a guide wire 236 which has been positioned with the aid of a guide plate 242 and a guide plate manipulator 244.

Once the guide wire is positioned, a combination reamer/drill device 130 is positioned with the guide bore 156 aligned with the guide wire 236. The combination reamer/drill device 130 is then moved toward the guide wire 236 and at block 246 the guide wire 236 is used to guide the combination reamer/drill device 130 to a location adjacent to the glenoid 238 of the scapula 240 as shown in FIG. 15.

At block 248, a combination power extension/anti-rotation handle 160 is coupled to the combination device 130 by first aligning the guide bore 216 (see FIG. 12) with the guide wire 236. The combination power extension/anti-rotation handle 160 is then moved over the guide wire 236 until the guide wire 236 extends through the guide bore 216 and into the lower receiving area 214. The proximal portion 162 is then pivoted with respect to the distal portion 164 as necessary to align the guide bore 186 with the guide wire 236. The combination power extension/anti-rotation handle 160 is then moved over the guide wire 236 until the power transfer portion 200 is adjacent to the drive section 132 of the combination reamer/drill device 130. If needed, the combination power extension/anti-rotation handle 160 is rotated on the guide wire 236 to rotationally align the shaped power transfer portion 200 with the shaped drive section 132 and the power transfer portion 200 is inserted into the drive section 132 resulting in the configuration of FIG. 16.

A rotary tool (not shown) is then coupled to the combination power extension/ anti-rotation handle 160 at block 250. Thus, the rotary tool is coupled to the power receiving portion 166 of the proximal portion 162 so as to be indirectly coupled to the combination reamer/drill device 130.

Power is then applied to the rotary tool causing the rotary tool to rotate the combination power extension/anti-rotation handle 160. Rotary force is transferred to the drive section 132 of the combination reamer/drill device 130 through the power transfer portion 164 (see FIG. 12). More specifically, torque is passed from the rotary tool to the power receiving portion 166. The tines 180, 182, 208, 210 are configured such that the torque received by the proximal portion 162 is transferred to the tines 208, 210 through the tines 180, 182. The torque is then transferred from the power transfer portion 200 to the drive section 132, causing the combination reamer/drill device 130 to rotate.

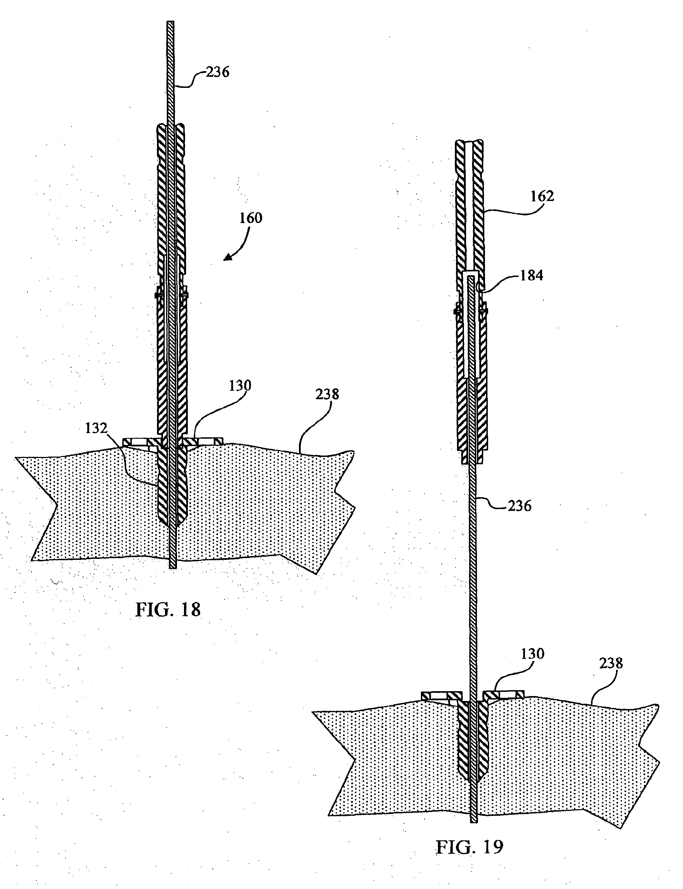

As the combination reamer/drill device 130 initially rotates about the guide wire 236, the drill section 136 contacts the glenoid 238 and begins to bore a hole in the glenoid 238. The reaming fins 140, however, are initially spaced apart from the glenoid 238 as shown in FIG. 17. Accordingly, no reaming occurs. As a hole is formed in the glenoid 238 by the drill section 136, the combination reamer/drill device 130 is guided by the guide wire 236 such that the reaming fins 140 come into contact with the glenoid 238 as shown in FIG. 18. Continued rotation of the combination reamer/drill device 130 with the rotary tool thus causes simultaneous reaming of the glenoid 238 with the reaming fins 140 and boring of the scapula 240 with the drill section 136 at block 252.

Once the glenoid 238 has been reamed to the desired depth, the power tool is deenergized and disconnected at block 254. The size of the drill section 132, both in length and diameter, is selected to be complimentary to the size of the centre peg 110 of the glenoid component 100. Thus, upon completion of the reaming, the bore formed by the drill section 132 is sized to receive the finned centre peg 110.

The combination power extension/anti-rotation handle 160 is then backed away from the combination reamer/drill device 130 along the guide wire 236 at block 256 until the end of the guide wire 126 is located within the receiving area 184 of the proximal portion 162 resulting in the configuration shown in FIG. 19. While the guide wire was within the guide bore 186 of the proximal portion 162, the proximal portion 162 was maintained in alignment with the distal portion 164 which enables smooth transfer of torque through the combination power extension/anti-rotation handle 160. Once the guide wire 236 is no longer within the guide bore 186, however, the proximal portion 162 may be pivoted with respect to the distal portion 164 to the configuration shown in FIG. 20 (block 258).

At block 260, the combination power extension/anti-rotation handle 160 is coupled to the combination device 130 substantially in the manner described above. However, since the guide wire 236 does not extend through the proximal portion 162, the proximal portion 162 may be used as a handle to rotate the coupled combination power extension/anti-rotation handle 160 and combination reamer/drill device 130 about an axis defined by the guide wire 236 as indicated by the arrow 262 of FIG. 21. The combination power extension/anti-rotation handle 160 is thus used to align a drill guide 146 in the combination reamer/drill device 130 with a desired location (block 264). Optionally, a handle similar to the handle shown attached to the guide plate manipulator 244 of FIG. 14 may be included in the kit. Such a handle may be removably coupled to the power receiving portion 166 of the proximal portion 162 to facilitate manipulation of the combination power extension/anti-rotation handle 160.

The ability to pivot the proximal portion 162 provides a surgeon with a relatively unobstructed view of the combination reamer/drill device 130. Accordingly, the surgeon may view the reamed surface of the glenoid 238 through the drill guides 146. This allows a surgeon to view the location in the scapula 240 at which the offset fixation pegs 112, 114, 116 of the glenoid component 100 will be anchored. In instruments according to this example in which the number and positioning of the drill guides 146 are complimentary to the number and positioning of the offset fixation pegs 112, 114, 116, the surgeon may orient the combination reamer/drill device 130 such that each of the drill guides 146 is aligned with portions of the scapula 240 that can provide a good anchor for the offset fixation pegs 112, 114, 116.

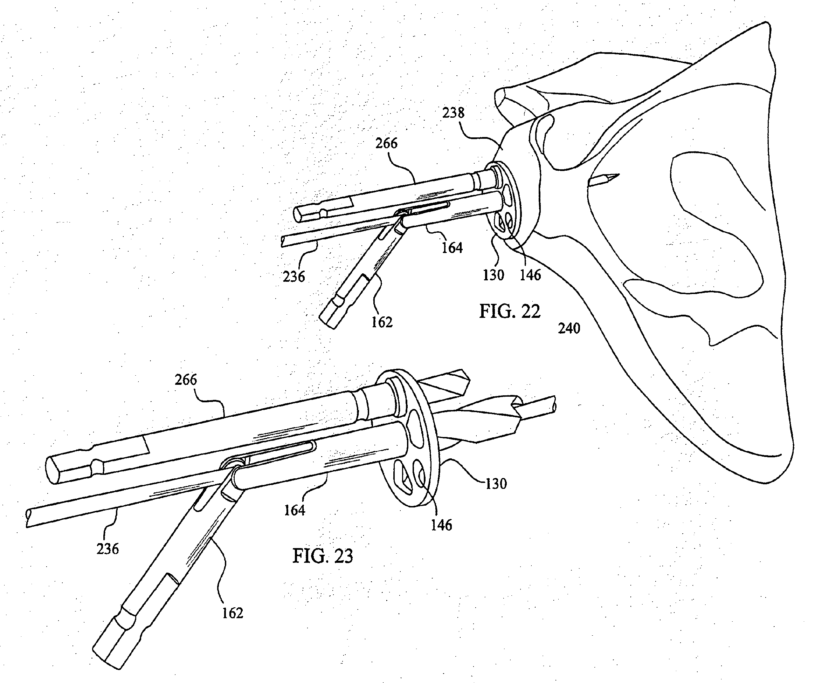

Once the combination reamer/drill device 130 is aligned at the block 264, a drill bit is inserted through one of the drill guides 146 to drill an additional bore at a location spaced apart from the first bore formed using the drill section 136 at block 230. By way of example, FIGs. 22 and 23 show a drill bit 266 positioned in a drill guide 146 of the combination device 130. The combination power extension/anti-rotation handle 160 may be used to steady the combination reamer/drill device 130 during the drilling process. The offset of the proximal portion 162 from the axis defined by the guide wire 236 results in a mechanical advantage in maintaining the combination reamer/drill device 130 at the desired orientation. Blocks 264, 268 may be repeated as desired to form additional holes.

Once all of the desired holes are formed, the combination reamer/drill device 130 is removed at block 270. The combination power extension/anti-rotation handle 160 may be used to aid in removal of the combination reamer/drill device 130. At block 272, the glenoid component is implanted. In this example, the glenoid component 100 has a lower bone contacting surface 106 shaped complimentary to the reaming cross-section of the reaming fins 140. Thus, in this example the lower bone contacting surface 106 is curved complimentary to the distal curve of the reaming fins 140. In other examples, the reaming fins 140 may be configured to produce a flat bottomed area if a glenoid component with a flat lower bone contacting surface is used. Accordingly, a kit can include different combination devices with differently shaped reaming cross-sections.

Moreover, while in the instrument shown in FIGs. 10 to 12 the proximal portion 162 is only pivotably connected to the distal portion 164, in another instrument the proximal portion is both pivotably and slidably connected to the distal portion. This can be accomplished by providing longitudinal slots connected to the bores 218, 220. In this instrument, once the proximal portion is aligned with the distal portion, the proximal portion may be moved toward the distal portion. Accordingly, in addition to torque being passed through the tines in the junction portion, additional features may be incorporated in the proximal and distal portions which are selectively interlocked. This arrangement allows for a more robust connection between the proximal portion and the distal portion which is useful in instruments in which weaker materials are desired to be used in forming a combination power extension/anti-rotation handle.

In yet another instrument, the guide bores in the proximal portion and the distal portion are positioned substantially immediately adjacent to one another when the proximal and distal portions are aligned. In this instrument, pivoting between the proximal and distal portions is enabled by moving the combination power extension/anti-rotation handle such that the guide wire does not extend into the guide bore in the proximal portion.

## Claims

1. An instrumentation kit for use in preparing a bone to receive a prosthetic component, comprising at least one first combination device (160) which includes a proximal portion (162) configured to couple with a torque providing device and a distal portion (164) configured to couple with a first instrument (130), in which:
the proximal portion has first and second tines (180, 182) at one end, and
the distal portion has first and second tines (208, 210) at one end,
the first tine (180) on the proximal portion being pivotally connected to the first tine (208) on the distal portion, and the second tine (182) on the proximal portion being pivotally connected to the second tine (210) on the distal portion so that the at least one first combination device can be pivoted between:
a first position in which the proximal portion and the distal portion are (i) longitudinally aligned and (ii) configured to transfer a torque received by the proximal portion to the distal portion, and
a second position in which the proximal portion and the distal position are (i) not longitudinally aligned and (ii) configured to transfer a torque received by the proximal portion to the distal portion,
**characterised in that** the kit includes a guide wire (236), and the proximal portion (162) of the first combination device has a first guide bore (186) extending through it and the distal portion (164) has a second guide bore (216) extending through it, the first guide bore being aligned with the second guide bore when the first combination device is in its first position allowing the guide wire to extend through the proximal and distal portions, and the first guide bore being not aligned with the second guide bore when the first combination device is in its second position allowing the guide wire to extend through the distal portion but not through the proximal portion.

2. The instrumentation kit of claim 1, in which:
the first and second tines (180, 182) on the proximal portion (162) define a first receiving area (184) between them,
the first and second tines (208, 210) on the distal portion (164) define a second receiving area (214) between them,
the first guide bore (186) extends from the proximal tip (188) of the proximal portion to the first receiving area, and
the second guide bore (216) extends from the distal end (202) of the distal portion to the second receiving area.

3. The instrumentation kit of claim 1, in which the said first instrument (130) is included in the kit and comprises:
a boring section (136) configured to rotationally form a first bore in a bone,
a drive section (132) operably connected to the boring section and configured to receive a rotational force,
a reaming section (140) positioned proximally from the boring section and operably connected to the drive section, the reaming section configured to rotationally ream a portion of the bone, and
at least one drill guide (146) configured to guide a drill bit and positioned so as to guide the drill bit to form a second bore in the bone at a location spaced apart from the first bore,
in which the reaming section and the boring section are positioned with respect to each other such that when the combination device is positioned against the bone and the rotational force is applied to the drive section, the boring section rotationally forms a portion of the first bore in the bone and the reaming section simultaneously rotationally reams a portion of the bone adjacent to the first bore.

4. The kit of claim 3, in which:
the first instrument (130) includes a third guide bore (156) extending from a distal tip (150) of the boring section (136) to the drive section (132), and
the third guide bore (156) is aligned with the second guide bore (216) when the distal portion (164) is coupled with the first instrument.

5. The kit of claim 3, which includes a second instrument, the second instrument comprising:
a boring section (136) configured to rotationally form a first bore in the bone,
a drive section (132) operably connected to the boring section and configured to receive a rotational force, and
a reaming section (140) positioned proximally from the boring section and operably connected to the drive section, the reaming section configured to rotationally ream a portion of the bone, in which the second instrument defines a reaming diameter different from a reaming diameter defined by the first instrument.

## Patentansprüche

1. Instrumentenset zur Nutzung in der Vorbereitung eines Knochens zum Aufnehmen einer prothetischen Komponente, umfassend zumindest ein erstes Kombinationsgerät (160), welches einen proximalen Abschnitt (162) umfasst, der dazu eingerichtet ist, mit einem drehmomentbereitstellenden Gerät zu koppeln und einem distalen Abschnitt (164) umfasst, der dazu eingerichtet ist, mit einem ersten Instrument (130) zu koppeln, in dem:
der proximale Abschnitt erste und zweite Zacken (180, 182) an einem Ende aufweist, und
der distale Abschnitt erste und zweite Zacken (208, 210) an einem Ende aufweist,
wobei die erste Zacke (180) des proximalen Abschnitts drehbar mit der ersten Zacke (208) auf dem distalen Abschnitt verbunden ist und die zweite Zacke (182) auf dem proximalen Abschnitt drehbar mit dem zweiten Zacken (210) auf dem distalen Abschnitt verbunden ist, so dass das zumindest eine erste Kombinationsgerät gedreht werden kann zwischen:
einer ersten Position, in der der proximale Abschnitt und der distale Abschnitt (i) longitudinal fluchtend sind und (ii) dazu eingerichtet sind, ein Drehmoment, welches von dem proximalen Abschnitt empfangen wird, auf den distalen Abschnitt zu übertragen, und
einer zweiten Position, in der der proximale Abschnitt und der distale Abschnitt (i) nicht longitudinal fluchtend sind und (ii) dazu eingerichtet sind, ein Drehmoment, welches von dem proximalen Abschnitt empfangen wird, auf den distalen Abschnitt zu übertragen,
**dadurch gekennzeichnet, dass** das Set einen Führungsdraht (236) beinhaltet und der proximale Abschnitt (162) des ersten Kombinationsgeräts eine erste Führungsbohrung (186) aufweist, welche sich durch den selbigen erstreckt, und der distale Abschnitt (164) eine zweite Führungsbohrung (216) aufweist, welche sich durch den selbigen erstreckt, wobei die erste Führungsbohrung mit der zweiten Führungsbohrung fluchtet, wenn das erste Kombinationsgerät in der ersten Position ist, was es dem Führungsdraht erlaubt, sich durch den proximalen und distalen Abschnitt zu erstrecken, und die erste Führungsbohrung nicht mit der zweiten Führungsbohrung fluchtet, wenn das erste Kombinationsgerät in der zweiten Position ist, was es dem Führungsdraht erlaubt, sich durch den distalen Abschnitt aber nicht durch den proximalen Abschnitt zu erstrecken.

2. Instrumentenset nach Anspruch 1, in dem:
die erste und zweite Zacke (180, 182) an dem proximalen Abschnitt (162) zwischen sich einen ersten Aufnahmebereich (184) definieren,
die erste und zweite Zacke (208, 210) an dem distalen Abschnitt (164) zwischen sich einen zweiten Aufnahmebereich (214) definieren,
wobei sich die erste Führungsbohrung (186) von der proximalen Spitze (188) des proximalen Abschnitts zum ersten Aufnahmebereich erstreckt, und
die zweite Führungsbohrung (216) sich von dem distalen Ende (202) des distalen Abschnitts zu dem zweiten Aufnahmebereich erstreckt.

3. Instrumentenset nach Anspruch 1, in dem das erste Instrument (130) in dem Set beinhaltet ist und es umfasst:
einen Bohrabschnitt (136), welcher dazu eingerichtet ist, durch Drehung eine erste Bohrung in einem Knochen zu formen,
einen Antriebsabschnitt (132), wirkverbunden mit dem Bohrabschnitt, und dazu eingerichtet, eine Rotationskraft zu empfangen,
einen Erweiterungsbohrungsabschnitt (140), welcher proximal von dem Bohrabschnitt positioniert ist und mit dem Antriebsabschnitt wirkverbunden ist steht, wobei der Erweiterungsbohrungsabschnitt dazu eingerichtet ist, einen Teil des Knochens durch Drehung abzunehmen, und
zumindest eine Bohrführung (146), welche dazu eingerichtet ist, eine Bohrspitze zu führen und so positioniert ist, dass die Bohrspitze geführt wird, eine zweite Bohrung in dem Knochen an einem von der ersten Bohrung beabstandeten Ort zu formen,
wobei der Erweiterungsbohrungsabschnitt und der Bohrabschnitt relativ zueinander so positioniert sind, dass, wenn das Kombinationsgerät an den Knochen positioniert ist und die Rotationskraft auf den Antriebsabschnitt angewandt wird, der Bohrabschnitt durch Drehung einen Teil der ersten Bohrung in dem Knochen formt und der Erweiterungsbohrungsabschnitt gleichzeitig durch Drehung einen Teil des Knochens, welcher an der ersten Bohrung anliegt, abnimmt.

4. Set nach Anspruch 3, in dem das erste Instrument (130) eine dritte Führungsbohrung (156) beinhaltet, welche sich von der distalen Spitze (150) des Bohrabschnitts (136) zu dem Antriebsabschnitt (132) erstreckt, und
die dritte Führungsbohrung (156) mit der zweiten Führungsbohrung (216) fluchtet, wenn der distale Abschnitt (164) mit dem ersten Instrument gekoppelt ist.

5. Set nach Anspruch 3, welches ein zweites Instrument beinhaltet, wobei das zweite Instrument aufweist:
einen Bohrabschnitt (136), welcher dazu eingerichtet ist, durch Drehung eine erste Bohrung in dem Knochen zu formen,
einen Antriebsabschnitt (132), welcher wirkverbunden mit dem Bohrabschnitt ist und dazu eingerichtet ist, eine Rotationskraft zu empfangen, und
einen Erweiterungsbohrungsabschnitt (140), welcher proximal von dem Bohrabschnitt positioniert ist und mit dem Antriebsabschnitt wirkverbunden ist, wobei der Erweiterungsbohrungsabschnitt dazu eingerichtet ist, einen Teil des Knochens durch Drehung abzunehmen, wobei das zweite Instrument einen Erweiterungsbohrungsdurchmesser definiert, welcher anders ist als ein Erweiterungsbohrungsdurchmesser, welcher von dem ersten Instrument definiert wird.

## Revendications

1. Kit d'instruments pour utilisation dans la préparation d'un os destiné à recevoir un composant prothétique, comprenant au moins un premier dispositif de combinaison (160) qui inclut une portion proximale (162) configurée pour être couplé à un dispositif fournisseur de couple et une portion distale (164) configurée pour être couplée à un premier instrument (130), dans lequel :
la portion proximale présente des première et deuxième dents (180, 182) à une extrémité, et
la portion distale présente des première et deuxième dents (208, 210) à une extrémité,
la première dent (180) sur la portion proximale étant connectée d'une manière pivotante à la première dent (208) sur la portion distale, et la deuxième dent (182) sur la portion proximale étant connectée d'une manière pivotante à la deuxième dent (210) sur la portion distale de sorte que le au moins un premier dispositif de combinaison peut être amené à pivoter entre :
une première position dans laquelle la portion proximale et la portion distale sont (i) alignées longitudinalement, et (ii) sont configurées pour transférer un couple reçu par la portion proximale à la portion distale, et
une deuxième position dans laquelle la portion proximale et la portion distale (i) ne sont pas alignées longitudinalement, et (ii) sont configurées pour transférer un couple reçu par la portion proximale à la portion distale,
**caractérisé en ce que** le kit inclut un fil de guidage (236), et la portion proximale (162) du premier dispositif de combinaison présente un premier perçage de guidage (186) s'étendant à travers celle-ci, et la portion distale (164) possède un deuxième perçage de guidage (216) s'étendant à travers celle-ci, le premier perçage de guidage étant aligné avec le deuxième perçage de guidage lorsque le premier dispositif de combinaison se trouve dans sa première position permettant au fil de guidage de s'étendre à travers les portions proximale et distale, et le premier perçage de guidage n'étant pas aligné avec le deuxième perçage de guidage lorsque le premier dispositif de combinaison se trouve dans sa seconde position permettant au fil de guidage de s'étendre à travers la portion distale mais non pas à travers la portion proximale.

2. Kit d'instruments selon la revendication 1, dans lequel :
les première et deuxième dents (180, 182) sur la portion proximale (162) définissent une première zone de réception (184) entre elles,
les première et deuxième dents (208, 210) sur la portion distale (164) définissent une deuxième zone de réception (214) entre elles,
le premier perçage de guidage (186) s'étend de la pointe proximale (188) de la portion proximale à la première zone de réception, et
le deuxième trou de perçage (216) s'étend de l'extrémité distale (202) de la portion distale à la deuxième zone de réception.

3. Kit d'instruments selon la revendication 1, dans lequel ledit premier instrument (130) est inclus dans le kit et comprend :
une section d'alésage (136) configurée pour forme d'une manière rotationnelle un premier perçage dans un os,
une section d'entraînement (132) fonctionnellement connectée à la section d'alésage et configurée pour recevoir une force de rotation,
une section d'alésage (140) positionnée d'une manière proximale de la section de perçage et fonctionnellement connectée à la section d'entraînement, la section d'alésage étant configurée pour aléser par rotation une portion de l'os, et
au moins un guide-foret (146) configuré pour guider une mèche de forage et positionné de façon à guider la mèche de forage pour former un deuxième trou dans l'os à un emplacement espacé du premier perçage,
où la section d'alésage et la section de perçage sont positionnées l'une par rapport à l'autre de telle sorte que lorsque le dispositif de combinaison est positionné contre l'os et que la force de rotation est appliquée à la section d'entraînement, la section de perçage forme par rotation une portion du premier perçage dans l'os, et la section d'alésage alèse simultanément par rotation une portion de l'os adjacente au premier perçage.

4. Kit selon la revendication 3, dans lequel :
le premier instrument (130) comprend un troisième perçage de guidage (156) s'étendant d'une pointe distale (150) de la section de perçage (136) à la section d'entraînement (132), et
le troisième perçage de guidage (156) est aligné avec le deuxième perçage de guidage (216) lorsque la portion distale (164) est couplée avec le premier instrument.

5. Kit selon la revendication 3, qui comprend un deuxième instrument, le deuxième instrument comprenant :
une section de perçage (136) configurée pour former par rotation un premier perçage dans l'os,
une section d'entraînement (132) fonctionnellement connectée à la section de perçage et configurée pour recevoir une force de rotation, et
une section d'alésage (140) positionnée d'une manière proximale de la section de perçage et fonctionnellement connectée à la section d'entraînement, la section d'alésage étant configurée pour aléser par rotation une portion de l'os, le deuxième instrument définissant un diamètre d'alésage différent d'un diamètre d'alésage défini par le premier instrument.
